# EUROPEAN PATENT APPLICATION

(11) **EP 3 736 763 A1**
(43) Date of publication of application: **11.11.2020**
(21) Application number: 18908993.1
(22) Date of filing: 22.10.2018
(51) Int. Cl.: G06Q 50/22, G16H 20/00

(54) **INFORMATION PROCESSING DEVICE, ANALYZER, AND INFORMATION PROCESSING SYSTEM**

(30) Priority: 07.03.2018 JP 2018040307
(71) Applicant: Sony Corporation, 108-0075 Tokyo (JP)
(72) Inventor: HIROBE, Keisuke, Tokyo 140-0002 (JP)
(74) Representative: D Young & Co LLP
(86) International application number: PCT/JP2018/039213
(87) International publication number: WO 2019/171651

(57) **Abstract**

It is desirable that a technology is provided capable of increasing user's consciousness of continuing activity.

An information processing device is provided including: a determination unit that determines an incentive to be provided to a user on the basis of activity information of the user or an analysis result of the activity information; and a providing unit that provides the incentive to the user.

## Description

### TECHNICAL FIELD

The present disclosure relates to an information processing device, an analysis device, and an information processing system.

### BACKGROUND ART

In recent years, a technology has been known for recording activity information of a user for maintaining health of the user, for example. For example, a technology has been disclosed for recording the activity information of the user and presenting a physical condition based on the activity information to the user (for example, see Patent Document 1). According to such a technology, the user can grasp how much activity is required for maintaining the health.

### CITATION LIST

### PATENT DOCUMENT

Patent Document 1: Japanese Patent Application Laid-Open No. 2016-24669

### SUMMARY OF THE INVENTION

### PROBLEMS TO BE SOLVED BY THE INVENTION

However, even if the user can grasp how much activity is required for maintaining the health, it may be difficult to continue the required activity. For example, even if a physical condition based on an activity status of the user is presented to the user, there is a case where user's consciousness of continuing the activity cannot be sufficiently increased. Thus, it is desirable that a technology is provided capable of increasing the user's consciousness of continuing the activity.

### SOLUTIONS TO PROBLEMS

According to the present disclosure, an information processing device is provided including: a determination unit that determines an incentive to be provided to a user on the basis of activity information of the user or an analysis result of the activity information; and a providing unit that provides the incentive to the user.

According to the present disclosure, an analysis device is provided including: an acquisition unit that acquires activity information of a user; and an analysis unit that obtains an analysis result by analyzing the activity information, in which on the basis of the analysis result, an incentive to be provided to the user is determined, and the incentive is provided to the user.

According to the present disclosure, an information processing system is provided including: a user terminal that measures activity information of a user; and a server including an acquisition unit that acquires activity information of a user, an analysis unit that obtains an analysis result by analyzing the activity information, a determination unit that determines an incentive to be provided to the user on the basis of the activity information or the analysis result, and a providing unit that provides the incentive to the user.

### EFFECTS OF THE INVENTION

As described above, according to the present disclosure, a technology is provided capable of increasing the user's consciousness of continuing the activity. Note that, the effect described above is not necessarily restrictive, and in addition to or instead of the effect described above, any of effects described in the present specification or other effects that can be grasped from the present specification may be achieved.

### BRIEF DESCRIPTION OF DRAWINGS

Fig. 1 is a diagram illustrating a configuration example of an information processing system according to an embodiment of the present disclosure.
Fig. 2 is a diagram illustrating an appearance example of a user terminal according to the embodiment of the present disclosure.
Fig. 3 is a diagram illustrating an appearance example of a band unit.
Fig. 4 is a diagram illustrating an appearance example of a core unit.
Fig. 5 is a diagram illustrating a functional configuration example of the user terminal according to the embodiment of the present disclosure.
Fig. 6 is a diagram illustrating a functional configuration example of a communication terminal according to the embodiment of the present disclosure.
Fig. 7 is a diagram illustrating a functional configuration example of a data acquisition server according to the embodiment of the present disclosure.
Fig. 8 is a diagram illustrating a configuration example of data stored by a storage unit of the data acquisition server.
Fig. 9 is a diagram illustrating a functional configuration example of a data update server according to the embodiment of the present disclosure.
Fig. 10 is a diagram illustrating a configuration example of data stored by a storage unit of the data update server.
Fig. 11 is a diagram for explaining details of functions of the information processing system according to the embodiment of the present disclosure.
Fig. 12 is a diagram for explaining details of functions of the information processing system according to the embodiment of the present disclosure.

### MODE FOR CARRYING OUT THE INVENTION

Hereinafter, preferred embodiments of the present disclosure will be described in detail with reference to the accompanying drawings. Note that, in the present specification and the drawings, components having substantially the same functional configuration are denoted by the same reference numerals, and redundant description will be omitted.

Furthermore, in the present specification and the drawings, a plurality of components having substantially the same or similar functional configuration may be distinguished from each other by adding different numerals after the same reference numerals. However, in a case where it is not necessary to particularly distinguish each of the plurality of components having substantially the same or similar functional configuration, only the same reference numeral is assigned. Furthermore, similar components in different embodiments may be distinguished by adding different alphabets after the same reference numerals. However, in a case where it is not necessary to particularly distinguish each of the similar components, only the same reference numeral is assigned.

Note that, the description will be made in the following order.

### 0. Overview

### 1. Details of embodiment

1.1. System configuration example
1.2. Configuration example of each unit of user terminal
1.3. Functional configuration example of user terminal
1.4. Functional configuration example of communication terminal
1.5. Functional configuration example of data acquisition server
1.6. Functional configuration example of data update server
1.7. Details of functions of information processing system

### 2. Conclusion

### <0. Overview>

In recent years, a technology has been known for recording activity information of a user for maintaining health of the user, for example. For example, a technology has been disclosed for recording the activity information of the user and presenting a physical condition based on the activity information to the user. According to such a technology, the user can grasp how much activity is required for maintaining the health. However, even if the user can grasp how much activity is required for maintaining the health, it may be difficult to continue the required activity. For example, even if a physical condition based on an activity status of the user is presented to the user, there is a case where user's consciousness of continuing the activity cannot be sufficiently increased. Thus, in the present specification, a technology will be mainly described capable of increasing the user's consciousness of continuing the activity.

In the above, the overview has been described of the embodiment of the present disclosure.

### <1. Details of embodiment>

Subsequently, details will be described of the embodiment of the present disclosure.

### [1.1. System configuration example]

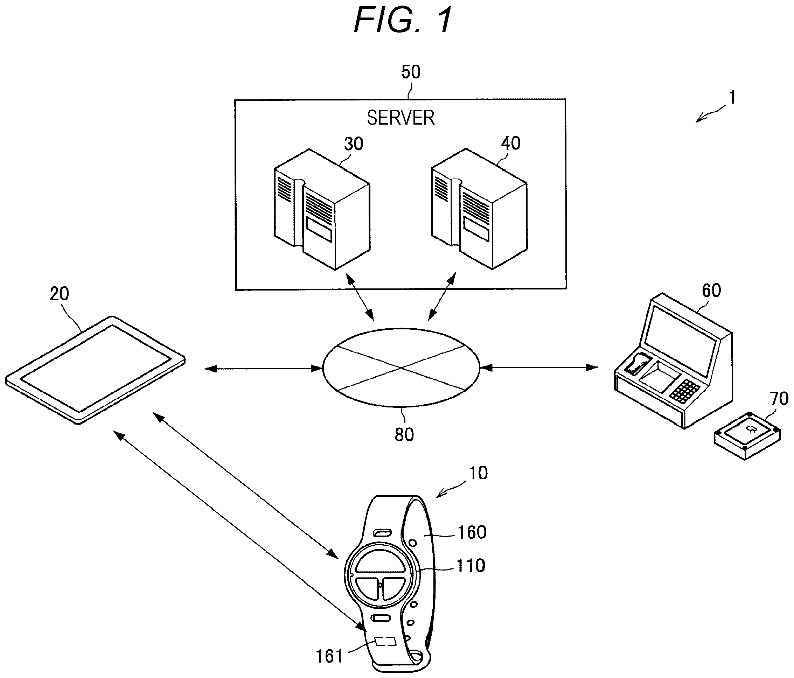

Fig. 1 is a diagram illustrating a configuration example of an information processing system according to an embodiment of the present disclosure. As illustrated in Fig. 1, an information processing system 1 includes a user terminal 10 (information processing device), a communication terminal 20, a server 50, a Point Of Sales (POS) terminal 60, and a card reading device 70.

Furthermore, in the embodiment of the present disclosure, a case is mainly assumed where the server 50 is separated into a data acquisition server 30 (analysis device) and a data update server 40 (information processing device). However, the server 50 may be integrally formed instead of being separated into the data acquisition server 30 and the data update server 40.

The communication terminal 20, the data acquisition server 30, the data update server 40, and the POS terminal 60 are connected to a network 80, and are able to communicate with each other via the network 80. As described later in detail, the user terminal 10 includes a core unit 110 and a band unit 160. The core unit 110 can communicate with the communication terminal 20 by short-range wireless communication. Furthermore, the band unit 160 includes an integrated circuit (IC) chip 161, and the IC chip 161 can communicate with the communication terminal 20 via an antenna by non-contact wireless communication. The POS terminal 60 is connected to the card reading device 70 by wire or wirelessly.

In the above, the configuration example has been described of the information processing system 1 according to the embodiment of the present disclosure.

### [1.2. Configuration example of each unit of user terminal]

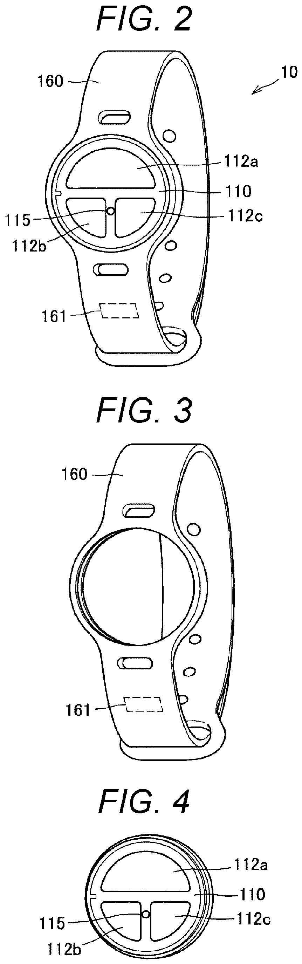

Subsequently, a configuration example will be described of each unit of the user terminal 10 according to the embodiment of the present disclosure. Fig. 2 is a diagram illustrating an appearance example of the user terminal 10 according to the embodiment of the present disclosure. As illustrated in Fig. 2, the core unit 110 can be attached to the band unit 160. That is, the user terminal 10 can take a state in which the core unit 110 and the band unit 160 are coupled to each other. Then, the band unit 160 can be worn on an arm of the user.

If the band unit 160 is worn on the arm of the user in the state in which the core unit 110 and the band unit 160 are coupled to each other, the user can carry the core unit 110 without holding the core unit 110 in a hand of the user. A description will be given later for an advantage that the state can be taken in which the core unit 110 and the band unit 160 are coupled to each other in this way. Furthermore, the core unit 110 includes a display unit 115, an alarm function button 112a, a meal/Bluetooth (registered trademark) (BT) button 112b, and a power button 112c.

The core unit 110 can record activity information. In the embodiment of the present disclosure, a case is assumed where the activity information includes the number of steps, sleep time, and meal time of the user. However, the activity information does not have to include all of the number of steps, sleep time, and meal time of the user, and may include at least one of the number of steps, sleep time, or meal time of the user.

The number of steps of the user can be recorded on the basis of acceleration detected by an acceleration sensor. Furthermore, the sleep time can be recorded on the basis of acceleration. That is, since bedtime and wake-up time of the user can be detected on the basis of acceleration, these two times can be recorded as the sleep time. Thus, if the band unit 160 is worn on the arm of the user in the state in which the core unit 110 and the band unit 160 are coupled to each other, the number of steps and the sleep time can be automatically acquired.

On the other hand, the meal time can be recorded on the basis of predetermined operation by the user. For example, in a case where predetermined operation (for example, pressing twice) is performed on the meal/BT button 112b by the user, the time at which the predetermined operation is performed can be recorded as the meal time. Note that, in the case where the predetermined operation (for example, pressing twice) is performed on the meal/BT button 112b, the display unit 115 may glow in a predetermined color (for example, orange or the like).

In addition, in the embodiment of the present disclosure, a case is assumed where the user terminal 10 has a waterproof function. In such a case, even if the user takes a bath or swims in a pool while wearing the user terminal 10, the user can avoid a failure of the user terminal 10. Furthermore, the core unit 110 incorporates a replaceable battery, and operates by power provided from the replaceable battery. As a result, it is not necessary to charge the battery.

Furthermore, long press operation (for example, operation of keeping the button pressed for several seconds, or the like) is performed by the user on the power button 112c, whereby on/off of the power source is switched. Furthermore, the long press operation (for example, the operation of keeping the button pressed for several seconds, or the like) is performed on the meal/BT button 112b, whereby a beacon radio wave for short-range wireless communication can be transmitted. The long press operation (for example, the operation of keeping the button pressed for several seconds) is performed on the alarm function button 112a, whereby on/off of an alarm is switched.

Fig. 3 is a diagram illustrating an appearance example of the band unit 160. As illustrated in Fig. 3, the band unit 160 can take a state of being separated from the core unit 110. If only the band unit 160 is worn on the arm of the user in a state in which the core unit 110 is separated from the band unit 160, the user can carry only the band unit 160 without carrying the core unit 110. Fig. 4 is a diagram illustrating an appearance example of the core unit 110. As illustrated in Fig. 4, the core unit 110 can take a state of being separated from the band unit 160.

In the above, the configuration example has been described of each unit of the user terminal 10 according to the embodiment of the present disclosure.

### [1.3. Functional configuration example of user terminal]

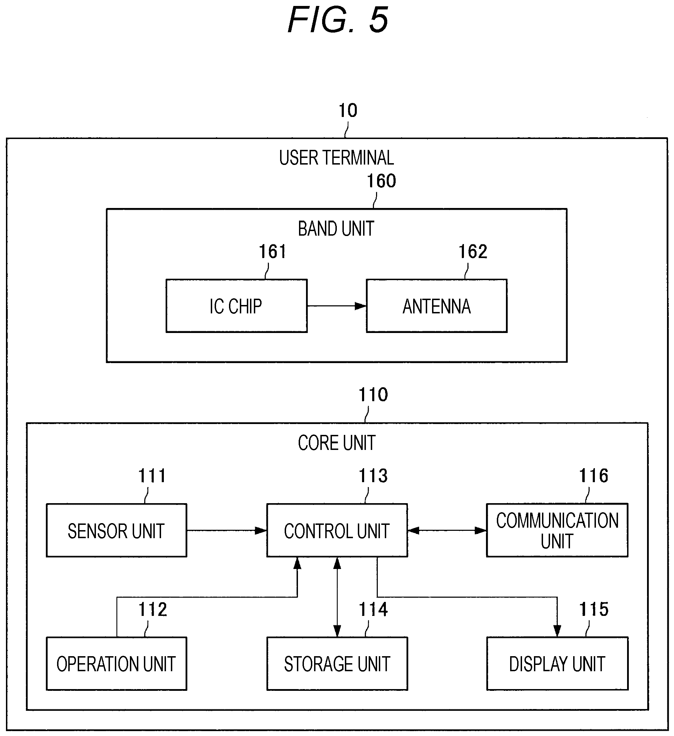

Subsequently, a functional configuration example will be described of the user terminal 10 according to the embodiment of the present disclosure. Fig. 5 is a diagram illustrating a functional configuration example of the user terminal 10 according to the embodiment of the present disclosure. As illustrated in Fig. 5, the user terminal 10 according to the embodiment of the present disclosure includes the core unit 110 and the band unit 160.

As illustrated in Fig. 5, the core unit 110 includes a sensor unit 111, an operation unit 112, a control unit 113, a storage unit 114, the display unit 115, and a communication unit 116. Hereinafter, these blocks included in the core unit 110 will be described.

The sensor unit 111 includes various sensors, and can acquire various sensor data by sensing with the sensors. In the embodiment of the present disclosure, an example will be mainly described in which the sensor unit 111 includes an acceleration sensor. However, types of the sensors included in the sensor unit 111 are not limited. Note that, acceleration acquired by the acceleration sensor can be used to measure the number of steps and sleep time of the user.

The operation unit 112 includes various input devices, and can acquire operation input by the user with the input devices. As described above, in the embodiment of the present disclosure, an example will be mainly described in which the operation unit 112 includes the alarm function button 112a, the meal/BT button 112b, and the power button 112c. However, types of the input devices included in the operation unit 112 are not limited.

The control unit 113 executes control of each unit of the core unit 110. Note that, the control unit 113 may include, for example, a central processing unit (CPU), and the like. In a case where the control unit 113 includes a processing device such as the CPU, the processing device may include an electronic circuit.

The storage unit 114 is a recording medium that stores a program executed by the control unit 113 and stores data necessary for executing the program. Furthermore, the storage unit 114 temporarily stores data for calculation by the control unit 113. The storage unit 114 may be a magnetic storage device, a semiconductor storage device, an optical storage device, or a magneto-optical storage device.

The display unit 115 outputs various types of information. In the embodiment of the present disclosure, a case is assumed where the display unit 115 includes a display capable of performing display visible to the user, and the display includes a light emitting diode (LED). However, the display may be a liquid crystal display or an organic electro-luminescence (EL) display. Furthermore, the core unit 110 may include an audio output device instead of the display unit 115 or together with the display unit 115, or may include a tactile presentation device that presents tactile sensation to the user.

The communication unit 116 includes a communication circuit and has a function of performing communication with another device. For example, the communication unit 116 has a function of acquiring data from the other device and providing data to the other device. In the embodiment of the present disclosure, a case is assumed where the communication unit 116 includes an antenna that performs communication with another device by short-range wireless communication by Bluetooth (registered trademark) or the like. For example, the communication unit 116 transmits an ID (hereinafter, also referred to as a "core ID") for identifying the core unit 110 (device) by short-range wireless communication to the other device via the antenna.

Furthermore, as illustrated in Fig. 5, the band unit 160 includes the IC chip 161 and an antenna 162. Hereinafter, these blocks included in the band unit 160 will be described.

Furthermore, the IC chip 161 includes a processing device and a memory. The processing device controls non-contact wireless communication by the antenna 162 with another device. At this time, the processing device outputs an ID (hereinafter, also referred to as a "card ID") for identifying the IC chip stored by the memory to the antenna 162, and controls transmission of the card ID to the other device by the antenna 162.

The antenna 162 performs communication with another device by non-contact wireless communication by FeliCa (registered trademark) or the like. Note that, the standard of non-contact wireless communication by the antenna 162 is not limited to FeliCa (registered trademark). For example, the antenna 162 transmits the card ID output from the IC chip 161 to the other device by non-contact wireless communication.

In the above, the functional configuration example has been described of the user terminal 10 according to the embodiment of the present disclosure.

### [1.4. Functional configuration example of communication terminal]

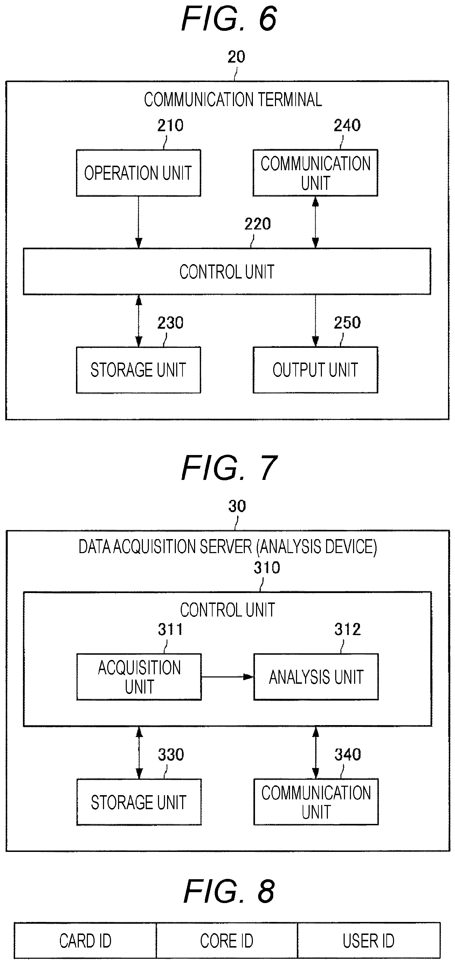

Subsequently, a functional configuration example will be described of the communication terminal 20 according to the embodiment of the present disclosure. Fig. 6 is a diagram illustrating a functional configuration example of the communication terminal 20 according to the embodiment of the present disclosure. As illustrated in Fig. 6, the communication terminal 20 according to the embodiment of the present disclosure includes an operation unit 210, a control unit 220, a storage unit 230, a communication unit 240, and an output unit 250.

The operation unit 210 includes various input devices, and can acquire operation input by the user with the input devices. In the embodiment of the present disclosure, a case is assumed where the communication terminal 20 is a tablet terminal including a touch panel. That is, in the embodiment of the present disclosure, a case is mainly assumed where the operation unit 210 includes the touch panel as an input device. However, a type of the input device is not limited to the touch panel. A type of the communication terminal 20 is not limited to the tablet terminal, and may be a notebook personal computer (PC) or a smartphone.

The control unit 220 executes control of each unit of the communication terminal 20. Note that, the control unit 220 may include, for example, a central processing unit (CPU), and the like. In a case where the control unit 220 includes a processing device such as the CPU, the processing device may include an electronic circuit.

The storage unit 230 is a recording medium that stores a program executed by the control unit 220 and stores data necessary for executing the program. Furthermore, the storage unit 230 temporarily stores data for calculation by the control unit 220. The storage unit 230 may be a magnetic storage device, a semiconductor storage device, an optical storage device, or a magneto-optical storage device.

The communication unit 240 includes a communication circuit and has a function of performing communication with another device. For example, the communication unit 240 has a function of acquiring data from the other device and providing data to the other device. In the embodiment of the present disclosure, a case is assumed where the communication unit 240 includes an antenna that performs communication with another device by non-contact wireless communication by FeliCa (registered trademark) or the like. However, the standard of non-contact wireless communication by the communication unit 240 is not limited to FeliCa (registered trademark). Furthermore, the communication unit 240 includes a communication interface for performing communication with another device via the network 80.

The output unit 250 outputs various types of information. In the embodiment of the present disclosure, a case is assumed where the output unit 250 includes a display capable of performing display visible to the user, and the display includes a light emitting diode (LED). However, the display may be a liquid crystal display or an organic electro-luminescence (EL) display. Alternatively, the output unit 250 may include a speaker instead of the display or together with the display, or may include a tactile presentation device that presents tactile sensation to the user.

In the above, the functional configuration example has been described of the communication terminal 20 according to the embodiment of the present disclosure.

### [1.5. Functional configuration example of data acquisition server]

Subsequently, a functional configuration example will be described of the data acquisition server 30 according to the embodiment of the present disclosure. Fig. 7 is a diagram illustrating a functional configuration example of the data acquisition server 30 according to the embodiment of the present disclosure. As illustrated in Fig. 7, the data acquisition server 30 according to the embodiment of the present disclosure includes a control unit 310, a storage unit 330, and a communication unit 340.

The control unit 310 executes control of each unit of the data acquisition server 30. Note that, the control unit 310 may include, for example, a central processing unit (CPU), and the like. In a case where the control unit 310 includes a processing device such as the CPU, the processing device may include an electronic circuit.

The storage unit 330 is a recording medium that stores a program executed by the control unit 310 and stores data necessary for executing the program. Here, an example will be described of data necessary for executing the program. Fig. 8 is a diagram illustrating a configuration example of data stored by the storage unit 330 of the data acquisition server 30. As illustrated in Fig. 8, the storage unit 330 stores information in which a card ID, a core ID, and an ID (hereinafter, also referred to as a "user ID") for identifying the user are associated with each other.

Returning to Fig. 7, the description will be continued. The storage unit 330 temporarily stores data for calculation by the control unit 310. The storage unit 330 may be a magnetic storage device, a semiconductor storage device, an optical storage device, or a magneto-optical storage device. An example will be described of data necessary for executing the program.

The communication unit 340 includes a communication circuit and has a function of performing communication with another device. For example, the communication unit 340 has a function of acquiring data from the other device and providing data to the other device. The communication unit 340 includes a communication interface for performing communication with another device via the network 80.

In the above, the functional configuration example has been described of the data acquisition server 30 according to the embodiment of the present disclosure.

### [1.6. Functional configuration example of data update server]

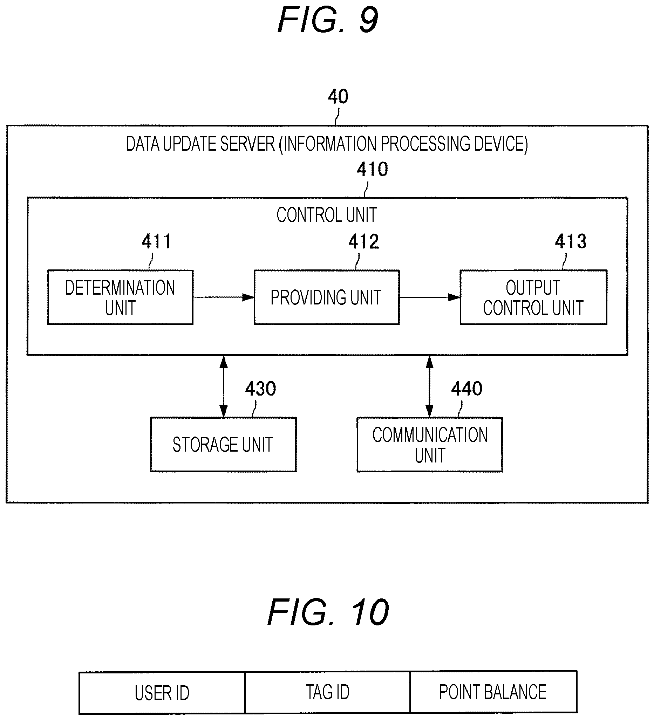

Subsequently, a functional configuration example will be described of the data update server 40 according to the embodiment of the present disclosure. Fig. 9 is a diagram illustrating a functional configuration example of the data update server 40 according to the embodiment of the present disclosure. As illustrated in Fig. 9, the data update server 40 according to the embodiment of the present disclosure includes a control unit 410, a storage unit 430, and a communication unit 440.

The control unit 410 executes control of each unit of the data update server 40. Note that, the control unit 410 may include, for example, a central processing unit (CPU), and the like. In a case where the control unit 410 includes a processing device such as the CPU, the processing device may include an electronic circuit.

The storage unit 430 is a recording medium that stores a program executed by the control unit 410 and stores data necessary for executing the program. Here, an example will be described of data necessary for executing the program. Fig. 10 is a diagram illustrating a configuration example of data stored by the storage unit 430 of the data update server 40. As illustrated in Fig. 10, the storage unit 430 stores information in which a user ID, a tag ID, and a point balance of electronic money (hereinafter, also simply referred to as a "point balance") held by the user are associated with each other.

Returning to Fig. 10, the description will be continued. The storage unit 430 temporarily stores data for calculation by the control unit 410. The storage unit 430 may be a magnetic storage device, a semiconductor storage device, an optical storage device, or a magneto-optical storage device.

The communication unit 440 includes a communication circuit and has a function of performing communication with another device. For example, the communication unit 440 has a function of acquiring data from the other device and providing data to the other device. The communication unit 440 includes a communication interface for performing communication with another device via the network 80.

In the above, the functional configuration example has been described of the data update server 40 according to the embodiment of the present disclosure.

### [1.7. Details of functions of information processing system]

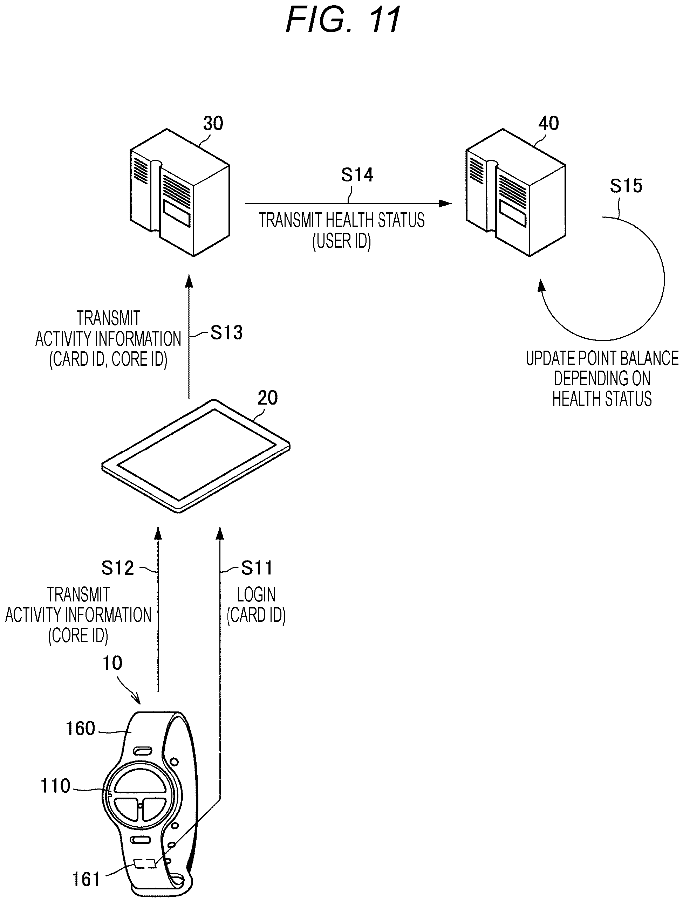

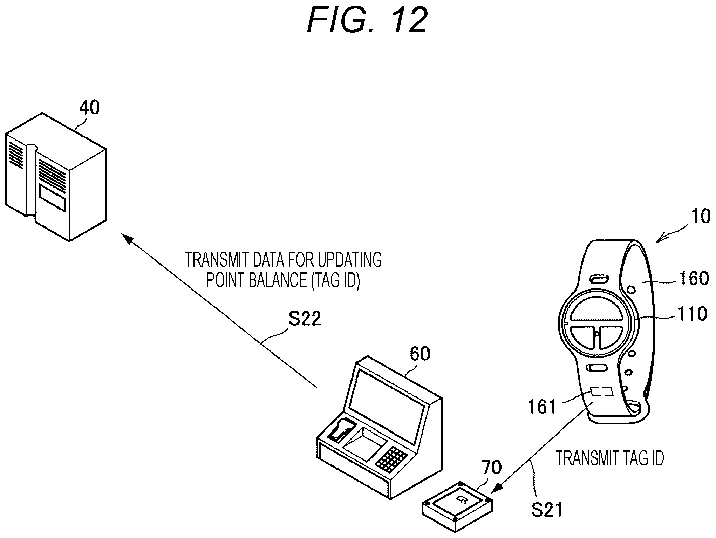

Subsequently, details will be described of functions of the information processing system 1 according to the embodiment of the present disclosure. Figs. 11 and 12 are diagrams for explaining the details of functions of the information processing system 1 according to the embodiment of the present disclosure. Note that, a case is assumed where the user is a member of a predetermined facility and the communication terminal 20 is a terminal installed in the predetermined facility. Here, the predetermined facility is not particularly limited.

As described above, the activity information of the user is recorded in the storage unit 114 of the core unit 110. For example, as illustrated in Fig. 11, a case is assumed where the user visits the predetermined facility while wearing on the arm the band unit 160 of the user terminal 10 in the state in which the core unit 110 and the band unit 160 are coupled to each other.

When the user terminal 10 is held over the communication terminal 20 by the user, the antenna 162 transmits the card ID recorded on the IC chip 161 to the communication terminal 20 by non-contact wireless communication. In the communication terminal 20, the communication unit 240 receives the card ID by non-contact wireless communication. As a result, login is performed by the user terminal 10 to the communication terminal 20 based on the card ID (S11). Note that, in the embodiment of the present disclosure, a case is assumed where not only one user terminal 10 but also a plurality of the user terminals 10 can log in to the communication terminal 20 at the same time.

On the other hand, in the core unit 110, the communication unit 116 transmits the activity information of the user and the core ID recorded in the storage unit 114 to the communication terminal 20 by short-range wireless communication (S12). In the communication terminal 20, the communication unit 240 receives the activity information and the core ID by short-range wireless communication. Moreover, the communication unit 240 transmits the activity information, the card ID, and the core ID to the data acquisition server 30 via the network 80 (S13).

In the data acquisition server 30, the communication unit 340 receives the activity information, the card ID, and the core ID from the communication terminal 20, and the acquisition unit 311 acquires the activity information, the card ID, and the core ID. The acquisition unit 311 acquires the user ID corresponding to the card ID and the core ID from the storage unit 330. Then, the analysis unit 312 obtains an analysis result of the activity information by analyzing the activity information. For example, the analysis result may be obtained by assigning the activity information to a predetermined arithmetic expression. The arithmetic expression may be prepared in advance or may be updated on the basis of a result of machine learning.

In the present embodiment, a case is mainly assumed where the analysis unit 312 of the data acquisition server 30 obtains a current health status of the user as the analysis result by analyzing the activity information. The health status may be a health score or the like that numerically indicates how healthy the user is. However, the analysis unit 312 may obtain a predicted future health status of the user by analyzing the activity information. In such a case, it is sufficient that the future health status is used instead of the current health status. For example, if machine learning is performed on a time change of the activity information and the health status, the future health status will be more accurately predicted on the basis of a result of the machine learning.

Alternatively, the analysis unit 312 does not have to analyze the activity information. In such a case, it is sufficient that the activity information itself acquired by the acquisition unit 311 is used instead of the health status. At this time, the data acquisition server 30 does not have to include the analysis unit 312. The communication unit 340 transmits the user ID and the health status obtained by the analysis unit 312 to the data update server 40 (S14).

In the data update server 40, the communication unit 440 receives the user ID and the health status from the data acquisition server 30. A determination unit 411 determines an incentive to be provided to the user on the basis of the health status received by the communication unit 440. Then, a providing unit 412 provides the incentive to the user.

In the embodiment of the present disclosure, a case is mainly assumed where the determination unit 411 determines a reward value to be provided to the user as the incentive. Then, a case is mainly assumed where the providing unit 412 provides the incentive by updating a balance held by the user on the basis of the reward value. If the balance is updated on the basis of the reward value (for example, if the reward value is added to the balance), the user can use the reward value to receive provision of a product or service, so that it is possible to increase the user's consciousness of continuing the activity.

Furthermore, in the embodiment of the present disclosure, a case is mainly assumed where the reward value is a point of electronic money. That is, a case is mainly assumed where the point balance of the user is updated on the basis of the point of electronic money (for example, the point of electronic money is added to the point balance of the user) (S15). However, the reward value may be an amount of cash. That is, an account balance of the user may be updated on the basis of the amount of cash (for example, the amount of cash may be added to the account balance of the user).

Alternatively, the determination unit 411 may determine a type of the product or a type of the service to be provided to the user as the incentive. Then, the providing unit 412 may provide the incentive by associating the type of the product or the type of the service with the user (user ID). If the association is performed between the type of the product or the type of the service and the user (user ID), the user can receive provision of the product or the service by using the association, so that it is possible to increase the user's consciousness of continuing the activity.

The association between the type of the product or the type of the service and the user (user ID) may be used in any way. For example, the providing unit 412 may transmit a coupon for the product or the service to a computer (not illustrated) of the user on the basis of the association. Alternatively, the providing unit 412 may execute printing of a paper coupon for the product or the service on the basis of the association. Then, the paper coupon may be mailed to an address of the user. In such a case, the user can receive provision of the product or the service by using the coupon.

Alternatively, the providing unit 412 may transmit information in which the type of the product or the type of the service is associated with the user ID to a terminal of a provider of the product or the service. In such a case, the terminal of the provider can receive the information in which the type of the product or the type of the service is associated with the user ID. Then, the provider confirms the information in which the type of the product or the type of the service is associated with the user ID, received by the terminal of the provider, thereby being able to provide the product or the service of the type associated with the user ID to a user identified by the user ID.

A method of determining the incentive is not particularly limited. For example, the determination unit 411 may determine the incentive in a case where the health status exceeds a threshold value, and does not have to determine the incentive in a case where the health status is less than or equal to the threshold value. For example, the determination unit 411 may determine the incentive in a case where the health status belongs to a normal range, and does not have to determine the incentive in a case where the health status does not belong to the normal range. At this time, the incentive may be determined in advance.

Alternatively, the determination unit 411 may determine a different incentive for each stage of the health status. For example, the determination unit 411 may determine the incentive so that the incentive is increased as the stage to which the health status belongs is more desirable. The stage and the incentive may be associated with each other in advance. Furthermore, in a case where an activity status itself is used instead of the health status, the incentive may be determined similarly. For example, in a case where the number of steps of the user exceeds a threshold value, the incentive may be determined, or in a case where the number of steps of the user belongs to a certain stage, the incentive corresponding to the stage may be determined.

As described above, when the incentive depending on the health status is provided to the user and the point balance is updated, the user can use more points from the updated point balance. Subsequently, a case will be described where the user uses points from the updated point balance.

Fig. 12 is a diagram for explaining the case where the user uses points from the updated point balance. In the example illustrated in Fig. 12, a case is assumed where the user visits a store where the points can be used while wearing on the arm the band unit 160 of the user terminal 10 in the state in which the core unit 110 and the band unit 160 are coupled to each other. However, the user may visit the store while wearing on the arm only the band unit 160. Note that, the user may be able to use the points at a place other than the store.

For example, when the user receives provision of the product or the service at the store (or the user orders the product or the service at the store) and an amount of payment for the provision of the product or the service is input to the POS terminal 60, the band unit 160 is held over the card reading device 70. As a result, in the band unit 160, the tag ID recorded on the IC chip 161 is transmitted by the antenna 162 by non-contact wireless communication (S21). The card reading device 70 receives the tag ID from the band unit 160.

When the tag ID is output from the card reading device 70 to the POS terminal 60, the POS terminal 60 sets points corresponding to the amount of payment as data for updating the point balance, and transmits the data for updating the point balance and the tag ID to the data update server 40 via the network 80 (S22). In the data update server 40, the communication unit 440 receives the data for updating the point balance and the tag ID.

An output control unit 413 updates the point balance associated with the tag ID by the storage unit 430 on the basis of the data for updating the point balance (for example, subtracts the data for updating the point balance from the point balance corresponding to the tag ID) . As a result, the user can receive provision of the product or the service by using the points. Furthermore, the user can receive the provision of the product or the service by using the points, whereby it is possible to increase the user's consciousness of continuing the activity. Note that, although not illustrated in Figs. 11 and 12, the information processing system 1 may include an electronic money server separately from the data update server 40. In such a case, in S22, the POS terminal 60 may directly access the electronic money server.

In the above, the details have been described of functions of the information processing system 1 according to the embodiment of the present disclosure.

### <2. Conclusion>

As described above, according to the embodiment of the present disclosure, the data update server 40 (information processing device) is provided including the determination unit 411 that determines the incentive to be provided to the user on the basis of the activity information of the user or the analysis result of the activity information, and the providing unit 412 that provides the incentive to the user. According to such a configuration, the user is provided with the incentive for performing the activity, so that it is possible to increase the user's consciousness of continuing the activity.

Furthermore, as described above, the core unit 110 and the band unit 160 can take a state of being coupled to each other. The band unit 160 can be used to receive provision of the product or the service (to purchase the product or the service) by using the point balance or the like. Thus, if the user wears on the arm the band unit 160 in the state in which the core unit 110 and the band unit 160 are coupled to each other to receive the provision of the product or the service, the core unit 110 is also carried naturally, so that an advantage is obtained that the activity information is easily acquired by the core unit 110.

Moreover, the core unit 110 and the band unit 160 are formed separately. As a result, processing of using the point balance or the like can be implemented by cooperation between the band unit 160 and the data update server 40. That is, the core unit 110 does not have to be involved in the processing of using the point balance or the like. As a result, even if the battery of the core unit 110 runs out, the processing of using the point balance (electronic money) can be implemented by the band unit 160. Furthermore, even if the battery of the core unit 110 runs out, the band unit 160 can be used as a membership card.

Furthermore, for example, in a case where electronic money of a smartphone is used, setting work of electronic money is required. On the other hand, in a case where electronic money of the user terminal 10 according to the embodiment of the present disclosure is used, the setting work of the electronic money does not have to be particularly performed. Thus, according to the embodiment of the present disclosure, since the electronic money can be used even if the setting work of the electronic money is not performed, the user terminal 10 can be implemented in which the electronic money is easily used even by a child or an elderly person.

In the above, the preferred embodiments of the present disclosure have been described in detail with reference to the accompanying drawings, but the technical scope of the present disclosure is not limited to such examples. It is apparent that a person having ordinary knowledge in the technical field of the present disclosure can come up with various changes or modifications within the scope of the technical idea described in the claims, and it is naturally understood that these also belong to the technical scope of the present disclosure.

For example, in the above, the case has been mainly described where the incentive is provided to the user on the basis of the activity information of the user (for example, the case where the point balance is updated). However, the incentive may be provided to the user on the basis of information other than the activity information. For example, in a case where the user holds the user terminal 10 over the communication terminal 20 and the user terminal 10 checks in an event, the incentive may be provided to the user. Alternatively, in a case where a predetermined application is used by the user terminal 10, the incentive may be provided to the user.

Alternatively, a case is also assumed where position information of the user terminal 10 can be detected on the basis of a beacon transmitted by the user terminal 10. In such a case, the incentive may be provided to the user in a case where the position information of the user terminal 10 moves to a predetermined place. As an example, a fact that the user leaves home can lead to health maintenance. Thus, in a case where it is determined that the user terminal 10 has moved out of the home on the basis of the position information of the user terminal 10 and position information of the user's home, the incentive may be provided to the user.

Furthermore, as long as the operation of the server 50 described above is implemented, the division of functions between the data acquisition server 30 and the data update server 40 is not particularly limited. As a specific example, in the above, the case has been mainly described where the data update server 40 determines whether or not the health status of the user exceeds the threshold value. However, the data acquisition server 30 may determine whether or not the health status of the user exceeds the threshold value. In such a case, in a case where the data acquisition server 30 determines that the health status of the user exceeds the threshold value, the data acquisition server 30 may transmit an incentive provision request and the user ID to the data update server 40.

Furthermore, in the above, the case has been mainly described where the tag ID corresponding to the user ID is acquired in the data update server 40 when the user ID corresponding to the card ID and the core ID is transmitted from the data acquisition server 30 to the data update server 40. However, the data acquisition server 30 may store information in which the card ID, the core ID, and the tag ID are associated with each other. In such a case, the tag ID corresponding to the card ID and the core ID may be transmitted directly from the data acquisition server 30 to the data update server 40.

Furthermore, in the above, the case has been mainly described where the data update server 40 stores the point balance. However, the point balance may be stored by a device different from the data update server 40. For example, the point balance may be stored by the IC chip 161 of the band unit 160 of the user terminal 10. That is, in a case where the incentive is determined by the data update server 40, the point balance recorded in the IC chip 161 may be updated on the basis of the points determined as the incentive.

Furthermore, the effects described in the present specification are merely illustrative or exemplary, and are not restrictive. That is, the technology according to the present disclosure can achieve other effects that are obvious to those skilled in the art from the description in the present specification, in addition to or instead of the effects described above.

Note that the following configurations also belong to the technical scope of the present disclosure.
(1) An information processing device including:
   a determination unit that determines an incentive to be provided to a user on the basis of activity information of the user or an analysis result of the activity information; and
   a providing unit that provides the incentive to the user.
(2) The information processing device according to (1), in which
   the determination unit determines a reward value to be provided to the user as the incentive.
(3) The information processing device according to (2), in which
   the reward value is a point of electronic money.
(4) The information processing device according to (2), in which
   the reward value is an amount of cash.
(5) The information processing device according to any one of (2) to (4), in which
   the providing unit provides the incentive by updating a balance held by the user on the basis of the reward value.
(6) The information processing device according to (1), in which
   the determination unit determines a type of a product or a type of service to be provided to the user as the incentive.
(7) The information processing device according to (6), in which
   the providing unit provides the incentive by associating the type of the product or the type of the service with the user.
(8) The information processing device according to any one of (1) to (7), in which
   the analysis result is a current health status, or a future health status predicted from the activity information.
(9) The information processing device according to any one of (1) to (8), in which
   the determination unit determines the incentive in a case where the activity information or the analysis result exceeds a threshold value.
(10) The information processing device according to any one of (1) to (9), in which
   the activity information includes at least one of the number of steps, sleep time, or meal time of the user.
(11) An analysis device including:
   an acquisition unit that acquires activity information of a user; and
   an analysis unit that obtains an analysis result by analyzing the activity information, in which
   on the basis of the analysis result, an incentive to be provided to the user is determined, and the incentive is provided to the user.
(12) An information processing system including:
   a user terminal that measures activity information of a user; and
   a server including
   an acquisition unit that acquires activity information of a user,
   an analysis unit that obtains an analysis result by analyzing the activity information,
   a determination unit that determines an incentive to be provided to the user on the basis of the activity information or the analysis result, and
   a providing unit that provides the incentive to the user.

### REFERENCE SIGNS LIST

- 1: Information processing system
- 10: User terminal
- 110: Core unit
- 111: Sensor unit
- 112: Operation unit
- 113: Control unit
- 114: Storage unit
- 115: Display unit
- 116: Communication unit
- 160: Band unit
- 161: IC chip
- 162: Antenna
- 20: Communication terminal
- 210: Operation unit
- 220: Control unit
- 230: Storage unit
- 240: Communication unit
- 250: Output unit
- 30: Data acquisition server (analysis device)
- 310: Control unit
- 311: Acquisition unit
- 312: Analysis unit
- 330: Storage unit
- 340: Communication unit
- 40: Data update server (information processing device)
- 410: Control unit
- 411: Determination unit
- 412: Providing unit
- 413: Output control unit
- 430: Storage unit
- 440: Communication unit
- 50: Server
- 60: POS terminal
- 70: Card reading device
- 80: Network

## Claims

1. An information processing device comprising:
a determination unit that determines an incentive to be provided to a user on a basis of activity information of the user or an analysis result of the activity information; and
a providing unit that provides the incentive to the user.

2. The information processing device according to claim 1, wherein
the determination unit determines a reward value to be provided to the user as the incentive.

3. The information processing device according to claim 2, wherein
the reward value is a point of electronic money.

4. The information processing device according to claim 2, wherein
the reward value is an amount of cash.

5. The information processing device according to claim 2, wherein
the providing unit provides the incentive by updating a balance held by the user on a basis of the reward value.

6. The information processing device according to claim 1, wherein
the determination unit determines a type of a product or a type of service to be provided to the user as the incentive.

7. The information processing device according to claim 6, wherein
the providing unit provides the incentive by associating the type of the product or the type of the service with the user.

8. The information processing device according to claim 1, wherein
the analysis result is a current health status, or a future health status predicted from the activity information.

9. The information processing device according to claim 1, wherein
the determination unit determines the incentive in a case where the activity information or the analysis result exceeds a threshold value.

10. The information processing device according to claim 1, wherein
the activity information includes at least one of a number of steps, sleep time, or meal time of the user.

11. An analysis device comprising:
an acquisition unit that acquires activity information of a user; and
an analysis unit that obtains an analysis result by analyzing the activity information, wherein
on a basis of the analysis result, an incentive to be provided to the user is determined, and the incentive is provided to the user.

12. An information processing system comprising:
a user terminal that measures activity information of a user; and
a server including
an acquisition unit that acquires activity information of a user,
an analysis unit that obtains an analysis result by analyzing the activity information,
a determination unit that determines an incentive to be provided to the user on a basis of the activity information or the analysis result, and
a providing unit that provides the incentive to the user.
